# EUROPÄISCHE PATENTSCHRIFT

(11) **EP 2 211 884 B1**
(45) Veröffentlichungstag und Bekanntmachung des Hinweises auf die Patenterteilung: **25.01.2012**
(21) Anmeldenummer: 08844034.2
(22) Anmeldetag: 30.10.2008
(51) Int. Cl.: A61K 36/85, A61K 36/35, A61K 36/185, A61K 36/51, A61K 36/70

(54) **HYDROLYSATE AUS PFLANZENEXTRAKTEN SOWIE DIESE ENTHALTENDES ANTIBAKTERIELLES MITTEL**
HYDROLYSATES MADE OF PLANT EXTRACTS AND ANTIBACTERIAL AGENT CONTAINING THE SAME
HYDROLYSATS D'EXTRAITS VÉGÉTAUX ET AGENT ANTI-BACTÉRIEN LES CONTENANT

(30) Priorität: 31.10.2007 DE 102007052223
(43) Veröffentlichungstag der Anmeldung: 04.08.2010
(73) Patentinhaber: BIONORICA SE, 92318 Neumarkt (DE)
(72) Erfinder: BONN, Günther, K., A-6170 Zirl (AT); STECHER, Günther, A-6091 Götzens (AT); POPP, Michael, A., 91207 Lauf (DE); MAYER, Robert, A-6236 Alpbach (AT)
(74) Vertreter: Simandi, Claus
(86) Internationale Anmeldenummer: PCT/EP2008/009171
(87) Internationale Veröffentlichungsnummer: WO 2009/056316

## Beschreibung

Die vorliegende Erfindung betrifft ein Hydrolysat aus wenigstens einem Extrakt aus wenigstens einem Pflanzenmaterial gemäß dem Oberbegriff des Anspruchs 1 sowie ein antibakterielles Mittel gemäß dem Oberbegriff das Anspruchs 18.

Extrakte aus den fünf heimischen Arzneipflanzen:
Rumicis herba (Rumex acetosa L., Rumex acetosella L., Rumex obtusifolius L., Rumex patientia L. und Rumex crispus L.);
Verbena officinalis L.;
Sambucus nigra L.;
Primula veris L. und Primula elatior (L.) Hill; und
Gentiana lutea L.
sind in Kombination als Sekretolytikum bei Infekten der oberen Atemwege und speziell bei Sinuisitiden unter der Marke SINUPRET^{®} (eingetragene Marke der BIONORICA) seit über 70 Jahren bekannt.

Jede Einzeldroge trägt dabei einen Anteil zur einzigartigen Wirksamkeit der Zusammensetzung bei:
Von Gentiana lutea (Gelber Enzian) wird in der Regel die Wurzel arzneilich verwendet. Unter den Inhaltsstoffen, welche eine schleimlösende Wirkung ausüben, findet man unter anderem verschiedene Secoiridoidglykoside.

Bei Rumicis herba (Sauerampferkraut) lösen die Wirkstoffe aus Blättern und Stengeln den Schleim, wirken entzündungshemmend und haben einen positiven Einfluß auf die körpereigenen Abwehrkräfte. Inhaltsstoffe umfassen z. B. Flavonoide, Oxalsäure und verschiedene Gerbstoffe.

Verbena officinalis (Eisenkraut) hat eine schleimlösende Wirkung und wirkt antiviral. Die Hauptinhaltsstoffe der arzneilich verwendeten Blätter und Stengel sind u.a. Iridoidglykoside, Zimtsäureglykoside und Flavonoide.

Von Sambucus nigra (Schwarzer Holunder) werden die Blüten, deren Inhaltsstoffe schleimlösend wirken, verwendet. Die Inhaltsstoffe umfassen z. B. verschiedene Flavonolglykoside und als Hauptwirkstoff Sambunigrin, ein cyanogenes Glykosid, welches antiviral wirkt.

Die Wirkstoffe aus Blüten und Kelch von Primula veris (Schlüsselblume) und/oder Primula elatior (L.) Hill (Hohe Schlüsselblume) bekämpfen Viren und wirken schleimlösend. Die Inhaltsstoffe umfassen z. B. Triterpensaponine sowie Phenolglykoside, wie Primulaverin. Primula veris bzw. Primula elatior wirkt als mildes Sekretolytikum und Expektorans bei der Behandlung von Atemwegserkrankungen.

Durch eine Kombination dieser fünf Heilpflanzen erhält man eine Zusammensetzung, mit der man eine für medizinische Zwecke ausreichende Wirkung erzielen kann. Die Zusammensetzung ist vorzugsweise bei Infektionen des Hals-Nasen-Ohren-Bereichs anzuwenden und eignet sich speziell für eine Behandlung von akuter und chronischer Sinusitis.

Sinusitiden können akut oder chronisch auftreten. Beide Formen treten häufig auf, wobei sich in drei von vier Fällen die Sinusitis als Folge einer Ausweitung der Schleimhautentzündung eines Schnupfens auf die Nasen-Nebenhöhlen entwickelt.

Die Nasen-Nebenhöhlen zählen zu den oberen Atemwegen. Die Atemwege reichen von der Nasen-Haupthöhle mit den verschiedenen Nebenhöhlen bis zu den Lungenbläschen. Zu den Nasen-Nebenhöhlen zählen die Stirnhöhlen, die Siebbeinzellen, die Keilbeinhöhle und die Kieferhöhlen. Sämtliche genannten Knochenhöhlen sind innen mit Schleimhaut ausgekleidet und münden über enge Öffnungen, die sogenannten Ostien, in die Nasenhaupthöhle.

An dem schützenden Schleim, mit der die Oberfläche der Atemwege überzogen ist, bleiben Schmutzpartikel und Krankheitserreger wie Viren und Bakterien, die mit der Atemluft eindringen, haften und werden von den im Schleim enthaltenen Abwehrstoffen angegriffen und unschädlich gemacht. Damit die Fremdkörper aus dem Körper ausgeschwemmt werden können, muß der Schleim mit Hilfe der Flimmerhärchen des Flimmerepithels in Richtung Rachen abtransportiert werden, wo er verschluckt werden kann.

Um infektbedingte Atemwegserkrankungen abwehren zu können, muss die Schleimhaut ungehindert über Schutz- und Reinigungsmechanismen verfügen können. Dazu ist es beim Abtransport des mit Erregern beladenen Schleims unabdingbar, dass die Flimmerhärchen ungehindert arbeiten können und durch ihre wellenförmigen Bewegungen den Schleim weitertransportieren und daß der Schleim selbst frisch und dünnflüssig ist.

Bei einem Infekt sind die Schutz- und Reinigungsmechanismen der Schleimhaut nicht mehr voll funktionsfähig.

Viren wie Rhinoviren, Adenoviren oder Coronaviren lösen Entzündungsreaktionen der Schleimhäute aus, wodurch die Schleimhaut anschwillt und verstärkt Schleim produziert. Dabei kommt es zuerst zu wäßrigem, anschließend zu zähflüssigem Schleimfluß. Im Laufe der Entzündung der Nasenschleimhaut können die Ostien der Nebenhöhlen zuschwellen und Schleim kann somit nicht mehr in die Nase abfließen. Es kommt zu einem Stau in den Nebenhöhlen. In dem zähen Schleim können sich die Flimmerhärchen nicht mehr bewegen und verkleben..Der Reinigungsmechanismus der Schleimhaut funktioniert nicht mehr.

In einer solchen Umgebung zähflüssigen Schleims fühlen sich die ubiquitär vorhandenen Bakterien besonders wohl. Der Schleim stellt einen optimalen Nährboden für Bakterien dar, auf dem sie sich schnell vermehren können. Die akute Sinusitis entsteht auf diese Weise in drei von vier Fällen als Folge eines zunächst einfachen Schnupfens.

Wenn diese ungünstigen Bedingungen, wie eine geschwollene Schleimhaut und durch zähflüssigen Schleim verklebte Flimmerhärchen, länger andauern, kann es zu einer chronischen Sinusitis kommen, bei der Schleimhaut und Flimmerepithel auf Dauer geschädigt werden können.

HNO-relevante Erreger, die sich im Schleim einnisten, sind beispielsweise Staphylococcus aureus, Staphylococcus epidermidis, Streptococcus pyogenes, Streptococcus pneumoniae oder Haemophilus influenzae.

Unter ihnen findet sich auch ein gegen Methicillin resistenter Staphylococcus aureus-Stamm, MRSA genannt. Gegen dieses Bakterium richten Standardantibiotika wie β-Lactam-Antibiotika, zum Beispiel Oxacillin, Penicillin und Amoxicillin, nichts mehr aus, da sich durch den allzu häufigen Einsatz von Antibiotika, bei denen die Erreger nicht vollständig abgetötet werden, Resistenzen entwickelt haben. Ein zusätzliches. Risiko stellen diese Keime auf chirurgischen Intensivstationen dar, wo sie zu Lungenentzündungen, Wundinfektionen und Blutvergiftungen führen können.

Die gemahlenen Rohdrogen sowie ethanolisch/wäßrige Auszüge bzw. daraus hergestellte Trockenauszügen (herstellbar z.B. durch Abziehen des Lösungs-/Extraktionsmittels unter vermindertem Druck) aus Gentiana lutea, Rumicis herba, Verbena officinalis, Sambucus nigra und Primula veris werden bereits in dem Arzneimittel Sinupret^{®} (als Extrakt: in Formulierung als Tropfen; und als Dragees, welche die gemahlenen Rohdrogen enthalten) der Firma BIONORICA als Sekretolytikum verwendet und hat sich durch seine ausschließlich auf pflanzlicher Basis beruhenden Heilkraft bewährt.

Die in Sinupret^{®} verwendeten Heilpflanzen werden sorgfältig ausgewählt, untersucht und weiterverarbeitet. Die gleichbleibende Qualität des Arzneimittels erreicht BIONORICA durch optimal erarbeitete Anzucht- und Erntestrategien und strengste Qualitätskontrolle.

Im Falle einer Verlegung der oberen Atemwege durch dickflüssigen Schleim, regen die Inhaltsstoffe der verwendeten Zusammensetzung (Gentiana lutea : Rumicis herba : Verbena officinalis : Sambucus nigra : Primula veris = 1:3:3:3:3) die Bildung von frischem, dünnflüssigen Schleim an. Dadurch kann sich der aufgestaute Schleim lösen und in Richtung Rachenraum abfließen. Die Entzündung, der Nasenschleimhaut geht dadurch zurück, wobei die Schleimhaut wieder abschwillt und die Nebenhöhlen sich öffnen. Sinupret^{®} bewirkt somit auf schonende Art und Weise die Wiederherstellung der Selbstreinigungskraft der Atemwege. Ein Kennzeichen von Sinupret^{®} ist seine gute Verträglichkeit, wobei mit der von BIONORICA ermittelten Dosierung selten Nebenwirkungen beim Patienten auftreten und keine Wechselwirkungen mit anderen Arzneimitteln bekannt sind.

Der Hauptwirkstoff in Sambucus nigra, das cyanogene Glykosid Sambunigrin, zeigt eine antivirale Wirkung, die sich darauf zurückführen lässt, daß es sich über die Spikes der Grippeviren, mit denen sie in die Zellen eindringen und Infektionen auslösen können, legt und diese so blockiert (Grabovac, A. und Ullmer, A., Österreichische Apotheker-Verlagsgesellschaft m.b.H., 2003).

Die deutsche Patentschrift DE10 2005 053 926 B3 beschreibt eine topische antibakterielle Wirkung der eingangs genannten Einzeldrogen sowie der Drogenmischung.

Diese Wirkung war für die Fachwelt überraschend, da man bislang immer glaubte, dass Extrakte aus Rumicis herba, Verbena officinalis, Sambucus nigra und Primula veris lediglich sekretolytische und keine antibakteriellen Wirkungen zeigen.

Darüber hinaus ist aus der deutschen Patentanmeldung DE 103 41 579 A1 von Extrakten aus Gentiana lutea bekannt, dass sie antibakterielle Wirkungen zeigen.

Zwar liegt in beiden Fällen ein klinisch hochinteressantes Wirkungsspektrum gegen eine Vielzahl von klinisch relevanten Keimen vor, jedoch sind die erforderlichen Dosen bisweilen unpraktisch zu handhaben.

Ausgehend vom Stand der Technik der DE10 2005 053 926 B3 Sinupret^{®}-Zusammensetzung ist es somit Aufgabe der vorliegenden Erfindung, ein Material zur Verfügung zu stellen, welches eine verbesserte antibakterielle Wirksamkeit aufweist.

Die Lösung der obigen Aufgabe erfolgt durch die kennzeichnenden Merkmale der Ansprüche 1 und 18.

Die Erfindung betrifft insbesondere ein Hydrolysat aus wenigstens einem Extrakt, welcher hergestellt ist durch Extraktion mittels Ethanol/Wasser aus getrocknetem Pflanzenmaterial aus:
a) wenigstens einer der Pflanzen, ausgewählt aus der Gruppe bestehend aus: Verbena officinalis L., Sambucus nigra L., Primula veris L., Primula elatior (L.) Hill und Gentiana lutea L.; sowie einer Mischung davon; oder
b) einer Mischung aus: Verbena officinalis L., Sambucus nigra L., Primula veris L., Gentiana lutea L. und Rumicis herba; und
anschließendem Entfernen des Ethanol/Wasser-Extraktionsmittels, wobei das Hydrolysat durch hydrolytische Behandlung mit einer Mineralsäure aus dem Extrakt erhältlich ist.

Ein bevorzugtes Hydrolysat ist dadurch gekennzeichnet, dass die Extrakte mittels eines Extraktionsmittels aus 50 Vol.-% Ethanol und 50 Vol.-% Wasser aus dem Pflanzenmaterial über 24h unter Rühren und anschließendem Vakuumverdampfen des Lösungsmittels herstellbar sind.

Eine weitere bevorzugte Ausführungsform der Erfindung liegt in einem Hydrolysat welches durch hydrolytisches Behandeln der Pflanzenextrakte mit Salzsäure als Mineralsäure, insbesondere mit Salzsäure einer Konzentration von 1 M bis 10 M , vorzugsweise 6 bis 9 M, insbesondere ca. 8 M bei 80°C bis 100°C, insbesondere ca. 90°C, für 30 min bis 120 min, insbesondere 40 min bis 60 min, bevorzugt ca. 45 min, erhältlich ist.

Es ist bevorzugt, die hydrolytische Behandlung der Extrakte in Gegenwart von Ethanol, insbesondere mit Wasser verdünntem Ethanol, vorzugsweise 50 Vol.-% Ethanol, durchzuführen.

Damit die Hydrolysate der vorliegenden Erfindung physiologisch gut verträglich sind, werden die Extrakte nach dem Säurebehandlungsschritt zur Trockene eingeengt, vorzugsweise in Wasser, einem Puffer oder in verdünntem Ethanol aufgenommen und gegebenenfalls mit einer pharmazeutisch akzeptablen Base neutralisiert. Hierzu kommt als Base z.B. NaOH, Na₂CO₃ oder Na₂HPO₄ in Betracht.

Bevorzugt wird das Hydrolysat aus einem Extrakt hergestellt, welcher aus folgenden Pflanzenbestandteilen stammt:
Rumicis herba (Rumex acetosa L., Rumex acetosella L., Rumex obtusifolius L., Rumex patientia L. und Rumex crispus L.): Blätter und Stengel
Verbena officinalis: Blätter und obere Stengelabschnitte
Sambucus nigra: Blüten
Primula veris und/oder Primula eliator: Blüten und Kelch
Gentiana lutea: Wurzel.

Besonders bevorzugt ist ein Hydrolysat aus einem Extrakt aus einer Mischung von: Gentiana lutea: Wurzel;
Rumicis herba: Blätter und Stengel;
Verbena officinalis: Blätter und obere Stengelabschnitte;
Sambucus nigra: Blüten; und
Primula veris: Blüten und Kelch;
besteht, wobei die einzelnen Drogen im zu hydrolysierenden Extrakt insbesondere im Massenverhältnis von 1:1:1:1:1 bis 1:3:3:3:3 vorliegen.

Es hat sich völlig überraschend herausgestellt, dass die erfindungsgemäßen Hydrolysate eine antibakterielle Wirkung aufweisen.

Die Hydrolysate der vorliegenden Erfindung weisen im Allgemeinen eine signifikante antibakterielle Wirkung auf, die in ihrem Ausmaß mit einer Antibiotikakontrolle aus Amoxicillin und Clavulansäure (Masseverhältnis 6:1) vergleichbar ist. Im Gegensatz hierzu zeigen nicht hydrolytisch behandelte reine Extrakte nur eine geringe bis gar keine antibakterielle Aktivität im verwendeten Testsystem.

Die Hydrolysate der vorliegenden Erfindung können verwendet werden zur Herstellung von Mitteln mit antibakterielle Wirkung gegen haut- und atemwegsrelevante Bakterien, insbesondere grampositiven Kokken, insbesondere Staphylococcus aureus, Staphylococcus epidermidis, Streptococcus pyogenes, Streptococcus pneumoniae, Streptococcus mutans und/oder gramnegativen Stäbchenbakterien, insbesondere Haemophilus influenzae.

Die Hydrolysate wurden im Rahmen der vorliegenden Erfindung gegen folgende haut- HNO- und atemwegsrelevante Erreger getestet und für wirksam befunden: Staphylococcus aureus (ATCC 25923), Staphylococcus epidermidis (ATCC 12228), Streptococcus pneumoniae (DSMZ 20566), Streptococcus pyogenes (DSMZ 20565), Streptococcus mutans (ATCC 35668), Haemophilus influenzae (DSMZ 4690), Klebsiella pneumoniae (ATCC 13883), und Enterococcus casseliflavus (VRE) (DSMZ 20680) sowie gegen die Darmbakterien Escherichia coli (ATCC 25922) Enterococcus faecalis (VRE) (ATCC 19433).

In vorteilhafter und an sich bekannter Weise können die Hydrolysate der vorliegenden Erfindung zur Herstellung eines antibakteriellen Mittels verwendet werden. Derartige antibakterielle Mittel können zum Schutz und/oder zur Behandlung von Infektionen oral oder topisch auf Haut und Schleimhäuten in Form einer ihrer Anwendung entsprechenden galenischen Formulierung eingesetzt werden.

Die galenischen Formulierungen der Hydrolysate/antibakteriellen Mittel der vorliegenden Erfindung sind dadurch gekennzeichnet, dass die orale Formulierungen wie Dragees, Tabletten, Filmtabletten, Pulver, Kapseln oder flüssige Verdünnungen, insbesondere Tropfen, Säfte oder Sirupe umfassen.

Bei Einsatz zur topischen Applikation eignen sich insbesondere Sprays, Salben, Emulsionen, Puder, Pulver, flüssige oder feste Präparate für die Inhalation, Kompressen, Wund- und Zahnfleischeinlagen, Tamponaden, Tonsillenpinsellösungen, Gurgellösungen oder Spüllösungen für Nase und Ohr.

Dabei sind die oben angesprochenen Tamponaden auch für zahnärztliche Anwendungen geeignet.

Zur Verwendung der erfindungsgemäßen Hydrolysate als Spüllösungen für Nase und Ohr werden diese vorteilhaft in Kombination mit physiologischen oder hyperosmolaren Konzentrationen von Salzen oder Salzgemischen, eingesetzt.

Für den Einsatz als antibakterielles Mittel hat sich herausgestellt, dass ein als Lyophilisat vorliegendes Präparat viele Vorteile hat, nämlich insbesondere bei der Lagerung und der Langzeitstabilität.

Die antibakteriellen Mittel der vorliegenden Erfindung können selbstverständlich die pharmazeutisch üblichen Hilfsstoffe enthalten.

Bei mikrobiologischen Untersuchungen am Institut für Analytische Chemie und Radiochemie der Universität Innsbruck hat sich überraschenderweise herausgestellt, dass die erfindungsgemäßen Hydrolysate eine breite, teilweise ausgeprägte antibakterielle Wirkung gegen haut-, atemwegs - und HNO-relevante Erreger zeigen, die in entsprechenden Testen auf ihre antibakterielle Wirkung erheblich stärker ausgeprägt waren als dies bei unhydrolysierten Extrakten der Fall war. So ergab sich beispielsweise in antibakteriellen Empfindlichkeitstestungen mit dem Agardiffusionstest nach Mueller-Hinton [Mueller, H.J. and Hinton, J. (1941): A proteinfree medium for primary isolation of the Gonococcus and Meningococcus. Proc. Soc. Expt. Biol. Med.; 48:330-333], dass von den fünf nicht hydrolysierten Einzeldrogenextrakten lediglich Rumicis herba und Primula veris gegen mehrere Erreger wirksam waren und die unhydrolysierte Mischung aus Gentiana lutea, Sambucus nigra, Verbena officinalis, Rumicis herba und Primula veris überraschenderweise im Agardiffusionstest praktisch keine antibakterielle Wirkung gegen das getestete Bakterienreferenzpanel zeigte. Interessanterweise fand sich derselbe Befund, dass sich die antibakterielle Wirkung jeweils gegen höchstens einen Keim richtete, auch für die unterschiedlichsten Kombinationen aus wenigstens drei Einzeldrogen aus den fünf genannten.

Dies ist in Analogie zur deutsche Patentschrift DE10 2005 053 926 B3, woraus hervorgeht, dass die antibakterielle Wirkung von unhydrolysierten Einzeldrogen oder von Kombinationen davon ebenfalls nur für einige wenige Keime zu beobachten ist. Hinsichtlich der Erreger, für die die nichthydrolysierten Extrakte eine Wirkung zeigen, sowie dem Ausmaß der Wirkung besteht eine weitgehende Übereinstimmung zwischen dem Stand der Technik und der vorliegenden Erfindung.

Umso überraschender war dann zunächst der Befund, dass sich nach Hydrolyse der Extrakte der Einzeldrogen deren antibakterielle Wirkungsprofile zum Teil umkehrten: So zeigte beispielsweise ein erfindungsgemäß hydrolysiertes Extrakt aus Rumicis herba praktisch keine antibakterielle Aktivität mehr während der unhydrolysierte Extrakt zuvor die ausgeprägteste Wirkung gegen die meisten getesteten Bakterienstämme zeigte.

Die größte Überraschung ist es jedoch, dass ein Hydrolysat aus einer Mischung aus Gentiana lutea, Sambucus nigra, Verbena officinalis, Primula veris und Rumicis herba eine signifikante antibakterielle Wirkung gegen sämtliche getesteten Bakterienstämme zeigt.

Die Hydrolysate und das antibakterielle Mittel der vorliegenden Erfindung können somit vorteilhaft bei der Behandlung von durch atemwegsrelevante-Erreger ausgelösten Infektionen eingesetzt werden. Die nicht nur schleimlösende und entzündungshemmende Wirkung der untersuchten Drogen und Drogenmischungen wird durch die zusätzliche antibakterielle Wirkung ergänzt, wodurch eine Infektion der oberen Atemwege nicht nur durch das Lösen des zähflüssigen, mit Erregern beladenen Schleims abgeschwächt wird, sondern durch Abtöten der bakteriellen Erreger vollständig zum Erliegen kommt.

Durch die zum Einsatz kommende medizinische Wirkung auf Basis der fünf Heilpflanzen, Gentiana lutea, Rumicis herba, Verbena officinalis, Sambucus nigra und Primula veris, wird ein von Sinusitis betroffener Patient auf schonende Art und Weise ohne synthetisch-chemische Komponenten behandelt. Das Präparat zeichnet sich zudem durch eine gute Verträglichkeit im Hinblick auf seine Wechselwirkungen mit anderen Medikamenten und im Hinblick auf die selten auftretenden Nebenwirkungen aus.

Das antibakterielle Mittel der vorliegenden Erfindung ist besonders gegen folgende Erreger wirksam, wobei sie speziell gegen grampositive Kokken wie Staphylococcus aureus, Staphylococcus epidermidis, Streptococcus pyogenes, Streptococcus pneumoniae, und Klebsiella gegen gramnegative Stäbchenbakterien wie Haemophilus influenzae, Pseudomonas aeruginosa sowie gegen Enterobacteriaceae faecalis (VRE), Enterobacteriaceae casseliflavus und E. coli antibakterielle Wirksamkeit zeigt.

Somit betrifft die vorliegende Erfindung auch die Verwendung der erfindungsgemäßen Hydrolysate und antibakteriellen Mittel zur Herstellung eines Arzneimittels zur Behandlung von durch atemwegs- und HNO-relevanten Erregern ausgelösten Infektionen im Atemwegs- und HNO-Bereich.

Weitere Vorteile und Merkmale der vorliegenden Erfindung ergeben sich aufgrund der Beschreibung von Ausführungsbeispielen.

### Beispiele

### Herstellung der Pflanzenextrakte

Die Einzeldrogen Gentiana lutea, Rumicis herba, Verbena officinalis, Sambucus nigra und Primula veris sowie Mischextrakte davon mit variabler Drogenkomposition (5-Pflanzen-Extrakt, 4-Pflanzen-Extrakt, 3-Pflanzen-Extrakt) wurden in 50 % EtOH/H₂O (v/v, ca. 1 g Pflanzenmaterial auf 20 ml Lösungsmittel) für 24 h unter Rühren bei Raumtemperatur extrahiert. Für die Mischextrakte bedeutet dies, dass von jeder Pflanze wie bereits angegeben ca. 1 g Material verwendet wurde, wobei ein solches Materialgemisch mit insgesamt 20 ml Lösungsmittel extrahiert wurde. Das Massenverhältnis der Einzeldrogen betrug 1:1:1:1:1, 1:1:1:1, 1:1:1.

### Herstellung der Hydrolysate

1,6 ml der oben beschriebenen Extrakte wurden mit 320 µl 25 % HCl (entspricht 8,1 mol/L) und 80 µl 50 % EtOH versetzt und für 45 Minuten bei 90 °C hydrolysiert.

Zum Vergleich wurde in einem zweiten Schritt die Hydrolyse mit 1 ml Extrakt unter Hinzugabe von 1 ml 25 % HCl unter denselben Bedingungen durchgeführt. Nach Eindampfen der Extrakte wurde der Rückstand in 1 ml sterilem Wasser aufgenommen und die so erhaltene Testlösung wurde auf deren antibakterielle Wirkung getestet.

Die Wirkung der Hydrolysate wurde mit der Wirkung der Einzel- oder Mischextrakte vor der Hydrolyse verglichen. Unhydrolysierte Extrakte lagen in 50% EtOH/H₂O (siehe oben) vor, wobei, gegebenenfalls durch Eindampfen die Konzentration der Inhaltsstoffe der unhydrolysierten und hydrolyiserten Extrakte zur Übereinstimmung gebracht wurde. Dadurch lagen bei gleichen eingesetzten Massen der Pflanzendrogen, die extrahierten Inhaltsstoffe jeweils im gleichen Volumen vor.

### Untersuchung der antibakteriellen Wirkung vor und nach Hydrolyse der Pflanzenextrakte

### Screening Verfahren:

Auf Müller Hinton Agar Platten bzw. Müller Hinton Agar Platten mit 5 % Hammelblut, die eine unbekannte Konzentration der zu testenden Bakterien enthielten, wurden 80 µl Testlösung (unhydrolysiert oder hydrolysiert) aufgetragen und bei 37 °C für 24 h inkubiert.

### Spiral Platter:

Eine Bakterienkolonie wurde in 5 ml CASO-Bouillon suspendiert und bei 37 °C für 24 h inkubiert.

Der Überstand wurde nach dem Zentrifugieren der Probe abgenommen, mit 0,9 % NaCl gewaschen und auf eine Konzentration von 10⁷ cfu/ml (colony forming unit per milliliter) verdünnt.

Ebenfalls 80 µl Testlösung (unhydrolysiert oder hydrolysiert) wurde 1:2, 1:20 und 1: 200 verdünnt und mit der Bakteriensuspension vermischt (für Pneumococcus und H. influenzae: 1:10, für die restlichen Pathogene 1:100). Als Positivkontrolle wurde 0,9 % NaCl verwendet.

Die Proben wurden mit einem Whitley Automatic Spiral Platter (WASP) nach 0, 4 und 8 Stunden plattiert und bei 37 °C für 24 Stunden inkubiert.

### Vor Hydrolyse:

### Screening Tests: Einzeldrogen /Mischextrakt vor Hydrolyse

**Tabelle 1** Ergebnisse der Untersuchung auf antibakterielle Wirkungen der jeweiligen Einzelextrakte *Gentiana lutea (Gentiana I.), Sambucus nigra (Sambucus n.), Verbena officinalis (Verbena o.), Rumex herba (Rumex h.)* und *Primula veris (Primula v.)* sowie eines Mischextraktes aus allen 5 Pflanzen (5 Pfl. Extrakt) gegen die Pathogene *Staphylococcus aureus (Staph. aureus) (A TCC 25923), Pseudomonas aeruginosa (P. aeruginosa)(ATCC 27853), Streptococcus pneumoniae (Pneumococcus)(DSMZ 20566), Streptococcus pyogenes (Strept. pyogenes)(DSMZ 20565), Klebsiella pneumoniae (Klebsiella) (A TCC 13883), Escherichia coli (E. coli) (A TCC 25922), Haemophilus influenzae (H. influenzae) (DSMZ 4690), Staphylococcus epidermidis (Staph. epidermidis) (A TCC 12228), Enterococcus faecalis (VRE) (Ent. faecatis (VRE) (ATCC 19433))* und *Enterococcus casseliflavus (VRE) (Ent. casseliflavus (VRE) (DSMZ 20680)).* +M = antibakterielle Aktivität auf Mueller Hinton Agar; +B = antibakterielle Aktivität auf Mueller Hinton Agar mit 5 % Hammelblut, + = Wirkung auf beiden Platten

| | *Gentiana I.* | *Sambucus n.* | *Verbena o.* | *Rumicis h.* | *Primula v.* | 5 Pfl. Extrakt |
|---|---|---|---|---|---|---|
| Staph. aureus | Ø | Ø | Ø | + | +M | Ø |
| P. aeruginosa | Ø | +B | Ø | +B | Ø | Ø |
| Pneumococcus | Ø | Ø | Ø | +M | +M | Ø |
| Strept. pyogenes | Ø | Ø | Ø | +M | +M | Ø |
| Klebsiella | Ø | Ø | Ø | Ø | Ø | Ø |
| E. coli | Ø | Ø | Ø | Ø | Ø | Ø |
| H. influenzae | Ø | Ø | Ø | Ø | Ø | Ø |
| Staph. epidermidis | Ø | Ø | Ø | + | Ø | Ø |
| Ent. faecalis (VRE) | Ø | Ø | Ø | + | Ø | Ø |
| Ent. casseliflavus (VRE) | Ø | Ø | Ø | +M | Ø | Ø |

### Spiral Platter: Rumicis herba, vor Hydrolyse

Tabelle 2 Quantifizierung der antibakterielle Wirkung von Rumicis herba sowie von Primula veris mittels Spiral Plattierung gegen die in Tabelle 1 genannten Pathogene. Die Proben wurden jeweils in 1:20 bzw. 1:200 Verdünnungen quantifiziert. ++++ = 10² cfu/ml nach 0 h, +++ = 10² cfu/ml nach 4 h, ++ = 10² cfu/ml nach 8 h, + = 10³-10⁴ cfu/ml nach 8 h, (+) = höhere Aktivität im Vergleich zur Kontrollgruppe

| | *Rumicis h.* | | *Primula v.* | |
|---|---|---|---|---|
| | 1:20 | 1-200 | 1:20 | 1-200 |
| Staph. aureus | +++ | + | ++ | (+) |
| P. aeruginosa | Ø | Ø | Ø | Ø |
| Pneumococcus | ++++ | ++++ | ++++ | ++++ |
| Strept. pyoqenes | ++++ | ++++ | ++++ | ++ |
| Klebsiella | Ø | Ø | Ø | Ø |
| E. coli | Ø | Ø | Ø | Ø |
| H. influenzae | Ø | Ø | Ø | Ø |
| Staph. epidermidis | ++ | ++ | Ø | Ø |
| Ent. faecalis (VRE) | ++++ | ++++ | Ø | Ø |
| Ent. casseliflavus (VRE) | ++++ | ++++ | Ø | Ø |

### Screening Tests: Pflanzengemische in verschiedenen Zusammensetzung, nicht hydrolysiert

**Tabelle 3** Aus den in Tabelle 1 genannten Einzeldrogen wurden 50 % ethanolische Mischextrakte bestehend aus jeweils 5, 4 bzw. 3 Pflanzen hergestellt und auf deren antibkaterielle Wirkung gegen die in Tabelle 1 genannten Pathogene in Screening Tests untersucht. *Gentiana lutea (G), Sambucus nigra (S), Verbena officinalis (V), Rumicis herba* (R) und *Primula veris (P).* + = Wirkung auf beiden Platten (Müllter Hinton Agar, Müller Hinton Agar mit 5 % Hammelblut), (+) = Wirkung auf einer Platte

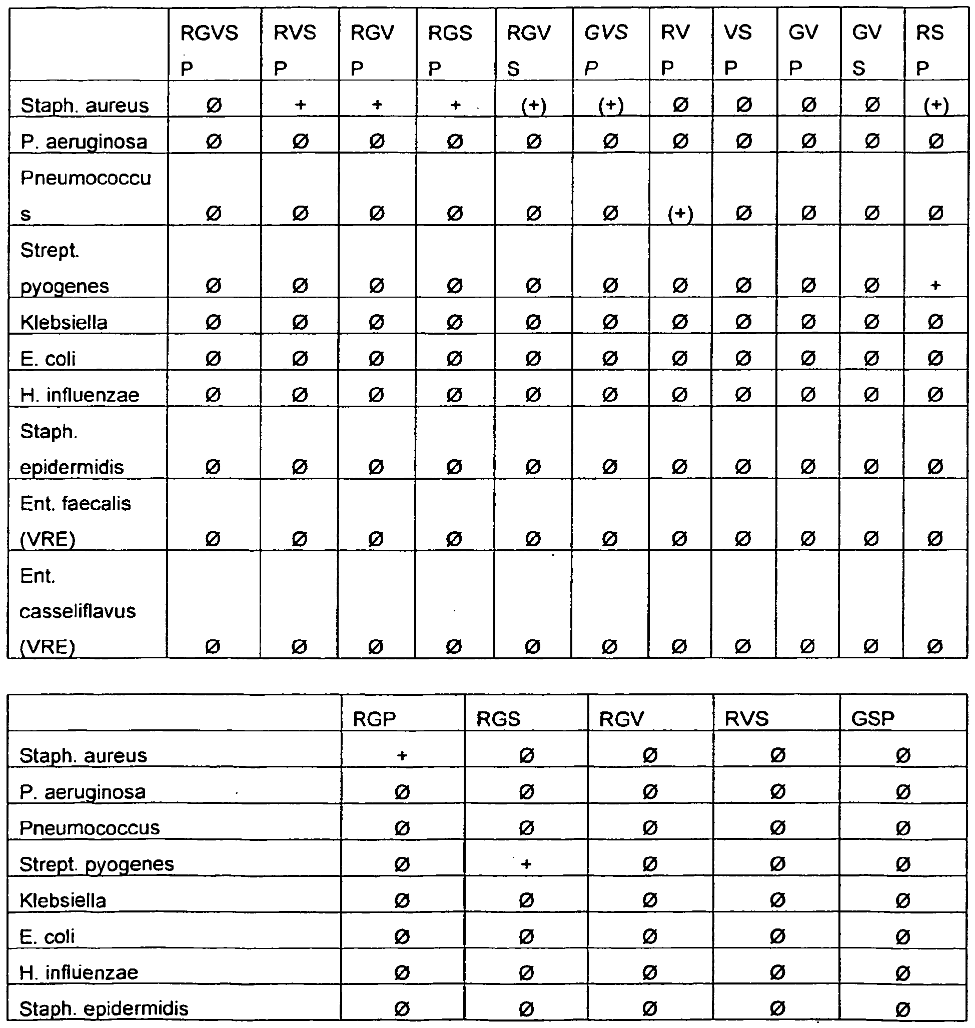

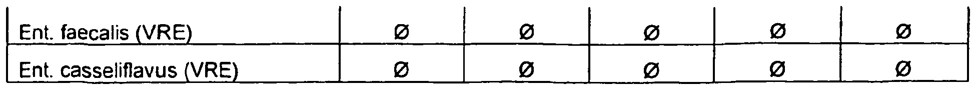

### Nach Hydrolyse:

### Screening Tests: Einzeldrogen /Mischextrakt nach Hydrolyse

**Tabelle 4** Die gewonnenen Extrakte der in Tabelle 1 genannten Pflanzendrogen sowie ein Mischextrakt bestehend aus allen fünf Pflanzen wurden mittels Salzsäure hydrolysiert und gegen die in Tabelle 1 genannten Pathogene auf antibakterielle Wirkung getestet. Die Extrakte wurden nach erfolgter Hydrolyse zur Trockene eingedampft und in sterilem Wasser gelöst. +M = antibakterielle Aktivität auf Mueller Hinton Agar; + = Wirkung auf beiden Platten (Müllter Hinton Agar, Müller Hinton Agar mit 5 % Hammelblut), (+) = Wirkung auf einer Platte

| | *Gentiana I*. | *Sambucu s n.* | *Verbena o.* | *Rumicis h.* | *Primula v.* | 5 Pfl. Extrakt |
|---|---|---|---|---|---|---|
| Staph. aureus | + | + | Ø | Ø | + | + |
| P. aeruginosa | + | (+) | Ø | Ø | + | + |
| Pneumococcus | + | + | + | Ø | + | + |
| Strept. pyogenes | + | + | + | Ø | + | + |
| Klebsiella | + | (+) | Ø | Ø | + | + |
| E. coli | + | Ø | Ø | Ø | + | + |
| H. influenzae | + | + | Ø | Ø | + | + |
| Staph. epidermidis | + | + | + | Ø | + | + |
| Ent. faecalis (VRE) | + | Ø | Ø | Ø | + | + |
| Ent. casseliflavus (VRE) | + | Ø | Ø | Ø | + | + |

### Spiral Platter: Quantifizierung der Einzeidrogen bzw. des 5-Pflanzenextraktes

**Tabelle 5** Quantifizierung der gewonnenen Hydrolysate der in Tabelle 1 genannten Pflanzendrogen sowie des 5-Pflanzenextraktes mittels Spiral Platter gegen die in Tabelle 1 genannten Pathogene. Alle Lösungen wurden in 1:20 Verdünnung gemessen. ++++ = 10² cfu/ml nach 0 h, +++ = 10² cfu/ml nach 4 h, ++ = 10² cfu/ml nach 8 h, + = 10³-10⁴ cfu/ml nach 8 h, (+) = höhere Aktivität im Vergleich zur Kontrollgruppe

| | *Gentiana I.* | *Sambucus n.* | *Verbena o.* | *Primula v.* | 5 Pfl. Extrakt |
|---|---|---|---|---|---|
| Staph. aureus | (+) | (+) | + | +++ | (+) |
| P. aeruginosa | ++++ | +++ | ++++ | ++++ | ++++ |
| Pneumococcus | +++ | + | (+) | ++++ | |
| Strept. pyogenes | ++++ | ++++ | ++++ | ++++ | ++++ |
| Klebsiella | +++ | ++ | ++ | +++ | +++ |
| E. coli | +++ | (+) | (+) | + | + |
| H. influenzae | ++++ | ++++ | ++++ | ++++ | ++++ |
| Staph. epidermidis | +++ | +++ | +++ | ++++ | +++ |
| Ent. faecalis (VRE) | +++ | ++ | + | +++ | +++ |
| Ent. casseliflavus (VRE) | +++ | ++ | +++ | +++ | +++ |

### Vergleich der Reproduzierbarkeit der antibakteriellen Wirkung

Tabelle 6 Studie zur Reproduzierbarkeit der antibakteriellen Wirksamkeit von mehreren Extrakten derselben Einzeldrogen *(Sambucus nigra, Gentiana lutea, Primula veris).* Quantifizierung mittels Spiral Platter in 1:20 Verdünnung der Probe. ++++ = 10² cfu/ml nach 0 h, +++ = 10² cfu/ml nach 4 h, ++ = 10² cfu/ml nach 8 h, + = 10³-10⁴ cfu/ml nach 8 h, (+) = höhere Aktivität im Vergleich zur Kontrollgruppe

| ***Sambucus n*.** | | | Extrakt 1 | Extrakt 2 | Extrakt 3 |
|---|---|---|---|---|---|
| Staph. aureus | | | (+) | (+) | (+) |
| P. aeruginosa | | | +++ | +++ | ++++ |
| Pneumococcus | | | + | ++ | ++ |
| Strept. pyogenes | | | ++++ | +++ | +++ |
| Klebsiella | | | ++ | (+) | +++ |
| E. coli | | | (+) | (+) | |
| H. influenzae | | | ++++ | ++++ | |
| Staph. epidermidis | | | +++ | ++ | ++++ |
| Ent. faecalis (VRE) | | | ++ | (+) | ++ |
| Ent. casseliflavus (VRE) | | | ++ | ++ | +++ |

| ***Gentiana I.*** | | Extrakt 1 | Extrakt 2 | Extrakt 3 | |
|---|---|---|---|---|---|
| Staph. aureus | | (+) | (+) | (+) | |
| P. aeruginosa | | ++++ | ++++ | ++++ | |
| Pneumococcus | | +++ | ++++ | ++++ | |
| Strept. pyogenes | | ++++ | ++++ | ++++ | |
| Klebsiella | | +++ | +++ | ++++ | |
| E. coli | | +++ | + | ++++ | |
| H. influenzae | | ++++ | ++++ | ++++ | |
| Staph. epidermidis | | +++ | +++ | ++++ | |
| Ent. faecalis (VRE) | | +++ | ++ | +++ | |
| Ent. casseliflavus (VRE) | | +++ | +++ | ++++ | |
| | | | | | |
| ***Primula v.*** | Extrakt | 1 Extrakt 2 | Extrakt 3 | Extrakt 4 | Extrakt 5 |
| Staph. aureus | +++ | (+) | ++ | (+) | + |
| P. aeruginosa | ++++ | +++ | ++++ | ++++ | ++++ |
| Pneumococcus | ++++ | (+) | +++ | +++ | +++ |
| Strept. pyogenes | ++++ | +++ | ++++ | ++++ | +++ |
| Klebsiella | +++ | (+) | ++++ | + | +++ |
| E. coli | + | (+) | +++ | ++ | + |
| H. influenzae | ++++ | +++ | ++++ | ++++ | ++++ |
| Staph. epidermidis | ++++ | (+) | +++ | +++ | +++ |
| Ent. faecalis (VRE) | +++ | (+) | +++ | + | + |
| Ent. casseliflavus (VRE) | +++ | ++ | +++ | +++ | ++ |

## Patentansprüche

1. Hydrolysat aus wenigstens einem Extrakt, welcher hergestellt ist durch Extraktion mittels Ethanol/Wasser aus getrocknetem Pflanzenmaterial aus:
a) wenigstens einer der Pflanzen, ausgewählt aus der Gruppe bestehend aus: Verbena officinalis L., Sambucus nigra L., Primula veris L., Primula elatior (L.) Hill und Gentiana lutea L.; sowie einer Mischung davon; oder
b) einer Mischung aus: Verbena officinalis L., Sambucus nigra L., Primula veris L., Gentiana lutea L. und Rumicis herba; und
anschließendem Entfernen des Ethanol/Wasser-Extraktionsmittels,
**dadurch gekennzeichnet, dass**
das Hydrolysat durch hydrolytische Behandlung mit einer Mineralsäure aus dem Extrakt erhältlich ist.

2. Hydrolysat nach Anspruch 1, **dadurch gekennzeichnet, dass** die Extrakte mittels eines Extraktionsmittels aus 50 Vol.-% Ethanol und 50 Vol.-% Wasser aus dem Pflanzenmaterial über 6h bis 36h, insbesondere 12 bis 30h, vorzugsweise ca.24h unter Rühren und gegebenenfalls anschließendem Vakuumverdampfen des Lösungsmittels herstellbar sind.

3. Hydrolysat nach Anspruch 1 oder 2, **dadurch gekennzeichnet, dass** es durch hydrolytisches Behandeln der Extrakte mit Salzsäure als Mineralsäure, insbesondere mit Salzsäure einer Konzentration von 1 M bis 10 M , vorzugsweise 6 bis 9 M, insbesondere ca. 8 M bei 80°C bis 100°C, insbesondere ca. 90°C, für 30 min bis 120 min, insbesondere 40 min bis 60 min, bevorzugt ca. 45 min, erhältlich ist.

4. Hydrolysat nach Anspruch 3, **dadurch gekennzeichnet, dass** die hydrolytische Behandlung der Extrakte in Gegenwart von Ethanol, insbesondere mit Wasser verdünntem Ethanol, vorzugsweise 50 Vol.-% Ethanol, durchgeführt wird.

5. Hydrolysat nach einem der vorhergehenden Ansprüche, **dadurch gekennzeichnet, dass** es zur Herstellung eines Trockenextraktes zur Trockene eingeengt wird und gegebenenfalls neutralisiert vorliegt.

6. Hydrolysat nach einem der vorhergehenden Ansprüche, **dadurch gekennzeichnet, dass** das Pflanzenmaterial zur Herstellung des Trockenextraktes aus folgenden Pflanzenbestandteilen stammt:
Rumicis herba (Rumex acetosa L., Rumex acetosella L., Rumex obtusifolius L.,
Rumex patientia L. und Rumex crispus L.): Blätter und Stengel
Verbena officinalis: Blätter und obere Stengelabschnitte
Sambucus nigra: Blüten
Primula veris und/oder Primula elatior: Blüten und Kelch
Gentiana lutea: Wurzel.

7. Hydrolysat nach Anspruch 6, **dadurch gekennzeichnet, dass** der
Trockenextrakt aus einer Mischung von Gentiana lutea: Wurzel,
Rumicis herba: Blätter und Stengel
Verbena officinalis: Blätter und obere Stengelabschnitte
Sambucus nigra: Blüten
Primula veris: Blüten und Kelch
besteht, wobei die einzelnen Drogen insbesondere im Massenverhältnis von 1:1:1:1:1 bis 1:3:3:3:3 vorliegen.

8. Hydrolysat nach einem der vorhergehenden Ansprüche, **dadurch gekennzeichnet, dass** es eine antibakterielle Wirkung aufweist.

9. Hydrolysat nach Anspruch 8, **dadurch gekennzeichnet, dass** sich die antibakterielle Wirkung gegen haut-, HNO- und atemwegsrelevante Bakterien, insbesondere grampositiven Kokken, insbesondere Staphylococcus aureus, Staphylococcus epidermidis, Streptococcus pyogenes, Streptococcus pneumoniae, Streptococcus mutans und/oder gramnegativen Stäbchenbakterien, insbesondere Haemophilus influenzae, richtet.

10. Hydrolysat nach Anspruch 9, **dadurch gekennzeichnet, dass** die Erreger ausgewählt werden aus Staphylococcus aureus (ATCC 25923), Staphylococcus epidermidis (ATCC 12228), Streptococcus pneumoniae (DSMZ 20566), Streptococcus pyogenes (DSMZ 20565), Haemophilus influenzae (DSMZ 4690); Enterococcus casseliflavus (DSMZ 20680); Pseudomonas aeruginosa (ATCC 27853); Klebsiella pneumoniae (ATCC 13883); E. coli und Enterococcus faecalis; sowie deren Mischungen.

11. Hydrolysat nach einem der vorhergehenden Ansprüche, **dadurch gekennzeichnet, dass** das es zum Schutz und/oder zur Behandlung von Infektionen oral oder topisch auf Haut und Schleimhäuten in Form einer galenischen Formulierung verabreichbar ist.

12. Hydrolysat nach Anspruch 11, **dadurch gekennzeichnet, dass** die oralen Formulierungen Dragees, Tabletten, Filmtabletten, Kapseln, flüssige Verdünnungen, insbesondere Tropfen, Säfte, Sirupe umfassen.

13. Hydrolysat nach Anspruch 11, **dadurch gekennzeichnet, dass** die Formulierungen zur topischen Applikation Sprays, Salben, Emulsionen, Puder, Pulver, flüssige oder feste Präparate für die Inhalation, Kompressen, Wund- und Zahnfleischeinlagen, Tamponaden, Tonsillenpinsellösungen, Gurgellösungen oder Spüllösungen für Nase und Ohr, umfassen.

14. Hydrolysat nach Anspruch 13 , **dadurch gekennzeichnet, dass** die Tamponaden auch für zahnärztliche Anwendungen geeignet sind.

15. Hydrolysat nach Anspruch 13, **dadurch gekennzeichnet, dass** die Spüllösungen für Nase und Ohr in Kombination mit physiologischen oder hyperosmolaren Konzentrationen von Salzen oder Salzgemischen, vorliegen.

16. Hydrolysat nach einem der Ansprüche 1 bis 15, **dadurch gekennzeichnet, dass** es als Lyophilisat vorliegt.

17. Antibakterielles Mittel, **dadurch gekennzeichnet, dass** es ein Hydrolysat gemäß wenigstens einem der Ansprüche 1 bis 16 enthält.

18. Antibakterielles Mittel nach einem der Anspruch 17, **dadurch gekennzeichnet**, das es die pharmazeutisch üblichen Hilfsstoffe enthält.

19. Antibakterielles Mittel nach Anspruch 17 oder 18, **dadurch gekennzeichnet**, das es als Retardformulierung vorliegt.

## Claims

1. A hydrolysate made of at least one extract, which is produced by extraction using ethanol/water from dried plant material:
a) at least one of the plants, selected from the group consisting of: Verbena officinalis L., Sambucus nigra L., Primula veris L., Primula elatior (L.) Hill und Gentiana lutea L.; and a mixture thereof; or
b) a mixture of: Verbena officinalis L., Sambucus nigra L., Primula veris L., Gentiana lutea L. and Rumicis herba; and
subsequent removal of the ethanol/water extraction agent,
**characterized in that**
the hydrolysate is obtainable from the extract by way of hydrolytic treatment using a mineral acid.

2. The hydrolysate according to claim 1, **characterized in that** the extracts can be produced from the plant material using an extraction agent made of 50% by volume ethanol and 50% by volume water over 6 hours to 36 hours, in particular 12 to 30 hours, preferably approximately 24 hours, while stirring and subsequent vacuum evaporation of the solvent.

3. The hydrolysate according to claim 1 or 2, **characterized in that** the hydrolysate can be obtained by the hydrolytic treatment of the plant extracts with hydrochloric acid as the mineral acid, in particular hydrochloric acid having a concentration of 1 M to 10 M, preferably 6 to 9 M, in particular approximately 8 M, at 80ºC to 100ºC, particularly approximately 90ºC, for 30 minutes to 120 minutes, in particular 40 minutes to 60 minutes, preferably approximately 45 minutes.

4. The hydrolysate according to claim 3, **characterized in that** the hydrolytic treatment of the extracts is carried out in the presence of ethanol, in particular ethanol diluted with water, preferably 50% by volume ethanol.

5. A hydrolysate according to any one of the preceding claims, **characterized in that** the hydrolysate is evaporated to dryness for producing a dry extract and optionally present in neutralized form.

6. A hydrolysate according to any one of the preceding claims, **characterized in that** the plant material for producing the dry extract stems from the following plant components:
Rumicis herba (Rumex acetosa L., Rumex acetosella L., Rumex obtusifolius L., Rumex patientia L. und Rumex crispus I.): leaves and stems
Verbena officinalis: leaves and upper stem sections
Sambucus nigra: blossoms
Primula veris and/or Primula eliator: blossoms and capsules
Gentiana lutea: roots.

7. The hydrolysate according to claim 6, **characterized in that**
the dry extract comprises a mixture of Gentiana lutea: roots,
Rumicis herba: leaves and stems
Verbena officinalis: leaves and upper stem sections
Sambucus nigra: blossoms
Primula veris: blossoms and capsules
wherein the individual drugs are present in particular in a mass ratio of 1:1:1:1:1 to 1:3:3:3:3.

8. A hydrolysate according to any one of the preceding claims, **characterized in that** it exhibits an antibacterial effect.

9. The hydrolysate according to claim 8, **characterized in that** the antibacterial effect is directed against skin, ENT and respiratory tract relevant bacteria, in particular gram positive cocci, especially Staphylococcus aureus, Staphylococcus epidermidis, Streptococcus pyogenes, Streptococcus pneumoniae, Streptococcus mutans and/or gram negative rod bacteria, in particular Haemophilus influenzae.

10. The hydrolysate according to claim 9, **characterized in that** the pathogens are selected from Staphylococcus aureus (ATCC 25923), Staphylococcus epidermidis (ATCC 12228), Streptococcus pneumoniae (DSMZ 20566), Streptococcus pyogenes (DSMZ 20565), Haemophilus influenzae (DSMZ 4690); Enterococcus casseliflavus (DSMZ 20680); Pseudomonas aeruginosa (ATCC 27853); Klebsiella pneumoniae (ATCC 13883); E. coli and Enterococcus faecalis; and mixtures thereof.

11. A hydrolysate according to any one of the preceding claims, **characterized in that** it can be used for the protection and/or treatment of infections orally or topically on the skin and mucous membranes in the form of a galenic formulation.

12. The hydrolysate according to claim 11, **characterized in that** the oral formulations comprises sugar-coated tablets, tablets, film-coated tablets, capsules, liquid dilutions, in particular drops, oral solutions and syrups.

13. The hydrolysate according to claim 11, **characterized in that** the formulations used for topical applications comprise sprays, ointments, emulsions, powders, liquid or solid preparations for inhalation, compresses, wound and gum dressings, tamponades, tonsil brush solutions, gargling solutions, or rinsing solutions for the nose and ears.

14. The hydrolysate according to claim 13, **characterized in that** the tamponades are also suited for dental applications.

15. The hydrolysate according to claim 13, **characterized in that** the rinsing solutions for the nose and ears are present in combination with physiological or hyperosmolar concentrations of salts or salt mixtures.

16. The hydrolysate according to any one of claims 1 to 15, **characterized in that** it is present as a lyophilisate.

17. An antibacterial agent, **characterized in that** it comprises a hydrolysate according to at least one of claims 1 to 16.

18. The antibacterial agent according to claim 17, **characterized in that** it comprises the pharmaceutically common adjuvants.

19. The antibacterial agent according to claim 17 or 18, **characterized in that** it is present as a retard formulation.

## Revendications

1. Hydrolysat fait d'au moins un extrait, qui est produit par extraction à l'aide d'éthanol/eau à partir de matière végétale séchée issue de :
a) au moins une des plantes, choisie dans le groupe consistant en : *Verbena officinalis L., Sambucus nigra L., Primula veris L., Primula elatior* (L.) Hill et *Gentiana lutea L. ;* et un mélange de celles-ci ; ou
b) un mélange de : *Verbena officinalis L., Sambucus nigra L., Primula veris L., Gentiana lutea L.* et *Rumicis herba ;* et
élimination ultérieure de l'agent d'extraction éthanol/eau,
**caractérisé par le fait que**
l'hydrolysat peut être obtenu à partir de l'extrait au moyen d'un traitement hydrolytique à l'aide d'un acide minéral.

2. Hydrolysat selon la revendication 1, **caractérisé par le fait que** les extraits peuvent être produits à partir de la matière végétale à l'aide d'un agent d'extraction fait de 50 % en volume d'éthanol et de 50 % en volume d'eau pendant 6 heures à 36 heures, en particulier 12 à 30 heures, de préférence environ 24 heures, tout en agitant, et par évaporation sous vide ultérieure du solvant.

3. Hydrolysat selon l'une des revendications 1 ou 2, **caractérisé par le fait que** l'hydrolysat peut être obtenu par le traitement hydrolytique des extraits végétaux par l'acide chlorhydrique en tant qu'acide minéral, en particulier l'acide chlorhydrique ayant une concentration de 1 M à 10 M, de préférence de 6 à 9 M, en particulier environ 8 M, à 80°C à 100°C, en particulier environ 90°C, pendant 30 minutes à 120 minutes, en particulier 40 minutes à 60 minutes, de préférence environ 45 minutes.

4. Hydrolysat selon la revendication 3, **caractérisé par le fait que** le traitement hydrolytique des extraits est réalisé en présence d'éthanol, en particulier d'éthanol dilué avec de l'eau, de préférence 50 % d'éthanol en volume.

5. Hydrolysat selon l'une quelconque des revendications précédentes, **caractérisé par le fait que** l'hydrolysat est évaporé jusqu'à l'état sec pour la production d'un extrait sec et facultativement présent sous forme neutralisée.

6. Hydrolysat selon l'une quelconque des revendications précédentes, **caractérisé par le fait que** la matière végétale pour la production de l'extrait sec a pour origine les composants végétaux suivants :
*Rumicis herba* (*Rumex acetosa L., Rumex acetosella L., Rumex obtusifolius L., Rumex patientia L.* et *Rumex crispus I.)* : feuilles et tiges
*Verbena officinalis* : feuilles et sections supérieures de tige
*Sambucus nigra* : bourgeons
*Primula veris* et/ou *Primula eliator* : bourgeons et capsules
*Gentiana lutea* : racines.

7. Hydrolysat selon la revendication 6, **caractérisé par le fait que** l'extrait sec comprend un
mélange de *Gentiana lutea* : racines,
*Rumicis herba* : feuilles et tiges
*Verbena officinalis* : feuilles et sections supérieures de tige
*Sambucus nigra* : bourgeons
*Primula veris* : bourgeons et capsules
dans lequel les médicaments individuels sont présents en particulier selon un rapport en masse de 1:1:1:1:1 à 1:3:3:3:3.

8. Hydrolysat selon l'une quelconque des revendications précédentes, **caractérisé par le fait qu'**il présente un effet antibactérien.

9. Hydrolysat selon la revendication 8, **caractérisé par le fait que** l'effet antibactérien est dirigé contre des bactéries appropriées de la peau, du système ORL et du tractus respiratoire, en particulier des cocci à Gram positif, en particulier *Staphylococcus aureus, Staphylococcus epidermidis, Streptococcus pyogenes, Streptococcus pneumoniae, Streptococcus mutans* et/ou des bactéries en batonnet à Gram négatif, en particulier *Haemophilus influenzae.*

10. Hydrolysat selon la revendication 9, **caractérisé par le fait que** les pathogènes sont choisis parmi *Staphylococcus aureus* (ATCC 25923), *Staphylococcus epidermidis* (ATCC 12228), *Streptococcus pneumoniae* (DSMZ 20566), *Streptococcus pyogenes* (DSMZ 20565), *Haemophilus influenzae* (DSMZ 4690) ; *Enterococcus casseliflavus* (DSMZ 20680) ; *Pseudomonas aeruginosa* (ATCC 27853) ; *Klebsiella pneumoniae* (ATCC 13883) ; *E. coli* et *Enterococcus faecalis ;* et leurs mélanges.

11. Hydrolysat selon l'une quelconque des revendications précédentes, **caractérisé par le fait qu'**il peut être utilisé pour la protection et/ou le traitement d'infections par voie orale ou par voie topique sur la peau et sur les membranes muqueuses sous la forme d'une formulation galénique.

12. Hydrolysat selon la revendication 11, **caractérisé par le fait que** les formulations orales comprennent des comprimés recouverts de sucre, des comprimés, des comprimés recouverts d'un film, des gélules, des dilutions liquides, en particulier des gouttes, des solutions orales et des sirops.

13. Hydrolysat selon la revendication 11, **caractérisé par le fait que** les formulations utilisées pour des applications topiques comprennent des vaporisations, des onguents, des émulsions, des poudres, des préparations liquides ou solides pour l'inhalation, des compresses, des pansements de cicatrisation et en gomme, des tampons, des solutions de brossage d'amygdales, des solutions de gargarisme ou des solutions de rinçage pour le nez et les oreilles.

14. Hydrolysat selon la revendication 13, **caractérisé par le fait que** les tampons sont également adaptés pour des applications dentaires.

15. Hydrolysat selon la revendication 13, **caractérisé par le fait que** les solutions de rinçage pour le nez et les oreilles sont présentes en combinaison à des concentrations physiologiques ou hyperosmolaires de sels ou de mélanges de sels.

16. Hydrolysat selon l'une quelconque des revendications 1 à 15, **caractérisé par le fait qu'**il est présent en tant que lyophilisat.

17. Agent antibactérien **caractérisé par le fait qu'**il comprend un hydrolysat selon au moins une des revendications 1 à 16.

18. Agent antibactérien selon la revendication 17, **caractérisé par le fait qu'**il comprend les adjuvants pharmaceutiquement classiques.

19. Agent antibactérien selon l'une des revendications 17 ou 18, **caractérisé par le fait qu'**il est présent sous forme de formulation retard.
